# EUROPÄISCHE PATENTSCHRIFT

(11) **EP 1 311 198 B1**
(45) Veröffentlichungstag und Bekanntmachung des Hinweises auf die Patenterteilung: **25.07.2007**
(21) Anmeldenummer: 00951183.3
(22) Anmeldetag: 24.08.2000
(51) Int. Cl.: A61B 17/70

(54) **VORRICHTUNG ZUR VERBINDUNG EINES KNOCHENFIXATIONSELEMENTES MIT EINEM LÄNGSSTAB**
DEVICE FOR CONNECTING A BONE FIXATION ELEMENT TO A LONGITUDINAL ROD
DISPOSITIF POUR RELIER UN ELEMENT DE FIXATION OSSEUSE A UNE TIGE OBLONGUE

(43) Veröffentlichungstag der Anmeldung: 21.05.2003
(73) Patentinhaber: Synthes GmbH, 4436 Oberdorf (CH)
(72) Erfinder: FRIGG, Robert, CH-2544 Bettlach (CH); DONATH, Raoul, D-79739 Grenzach-Wyhlen (DE)
(74) Vertreter: Lusuardi, Werther
(86) Internationale Anmeldenummer: PCT/CH2000/000449
(87) Internationale Veröffentlichungsnummer: WO 2002/015806

(56) Entgegenhaltungen:
- WO-A-00/15125
- FR-A- 2 794 962
- US-A- 5 474 551
- US-A- 5 487 744

## Beschreibung

Die Erfindung bezieht sich auf eine Vorrichtung zur Verbindung eines Knochenfixationselementes mit einem Längsstab gemäss dem Oberbegriff des Patentanspruchs 1.

Bei der posterioren Versorgung von Wirbelsäulen-Deformitäten werden Pedikelschrauben oder Haken verwendet, um Wirbelkörper mit einem Längsstab zu verbinden. Der Längsstab wird vorgeformt, um die Deformation der Wirbelsäule zu kompensieren. Diese Technik hat sich bewährt und gilt heute als Stand der Technik.

Trotz neuen Verbindungstechniken, wie z.B. polyaxial gelagerten Backen zur Schrauben-Längsstabverbindung, ist die intraoperative Verbindung der beiden Elemente, speziell bei starker Deformation der Wirbelsäule, problematisch. Der Grund dafür ist die definierte Distanz zwischen Pedikelschraubenkopf und Längsträger. Das heisst, die polyaxiale Lagerung des Schraubenkopfes in der Verbindungsbacke erlaubt nur eine variable Schraubenrichtung, jedoch keine variable Distanz zwischen Schraubenkopf und dem Längsträger. Diese variable Distanz zwischen dem Schraubenkopf oder Hakenende und dem Längsträger wäre oft wünschenswert, da eine 100%-ige Ausrichtung der Wirbelsäule auf den Längsstab nicht immer erreicht werden kann. Es sind Systeme bekannt, die eine variable Distanz zwischen Pedikelschrauben oder Haken zum Längsträger zulassen.

Eine solche Vorrichtung zur Verbindung von Pedikelschrauben, welche in die Pedikel an Wirbelkörpern einschraubbar sind, und einem Längsstab wird beispielsweise in der US 5,474,551 FINN offenbart. Das Verbindungselement umfasst einen Längsstabverbinder zur Verbindung eines Querverbinders mit dem Längsstab, wobei der Querverbinder relativ zum Längsstab in den senkrecht zueinander stehenden Achsrichtungen der Längsstabachse und der Querverbinderachse verschiebbar ist. Die Pedikelschraube ist polyaxial schwenkbar mit dem Querverbinder verbunden und so am Querverbinder angeordnet, dass die Längsachse der Pedikelschraube in ihrer nicht ausgeschwenkten Lage senkrecht auf der Längsstabachse und senkrecht auf der Querverbinderachse steht. Nachteilig an dieser bekannten Vorrichtung ist, dass die Distanz der Pedikelschraube oder des Pedikelhakens zum Längsträger nur unilateral variabel ist. Bei dieser unilateral variablen Backe muss die Lage des Längsstabes geplant medial oder lateral der Pedikelschraube oder des Pedikelhakens liegen. Das hat den Nachteil, dass die Distanz einzelner Pedikelschrauben oder Pedikelhaken zum Längsstab beträchtlich gross sein kann, um eine Korrektur bei den anderen Pedikelschrauben oder Pedikelhaken zu ermöglichen.

Hier will die Erfindung Abhilfe schaffen. Der Erfindung liegt die Aufgabe zugrunde, eine Vorrichtung zur Verbindung einer Pedikelschraube oder eines Pedikelhakens mit einem Längsträger zu schaffen, welche einerseits eine polyaxiale Lagerung der Pedikelschraube oder des Pedikelhakens ermöglicht und andererseits eine drehbare und in jeder Position fixierbare Verbindung zwischen dem Querverbinder und dem Längsstab gestattet.

Die Erfindung löst die gestellte Aufgabe mit einer Vorrichtung zur Verbindung eines Knochenfixationselementes mit einem Längsstab, welche die Merkmale des Anspruchs 1 aufweist.

Die erfindungsgemässe Vorrichtung umfasst im wesentlichen einen plattenförmigen Querverbinder mit einer Oberseite zur Anlage an den Längsstab, einer Unterseite und einen den Querverbinder von der Oberseite zur Unterseite konzentrisch zu einer ersten Achse durchdringenden Hohlraum, welcher zur Aufnahme einer radial elastisch deformierbaren Klemmbacke dient. Durch diese elastisch deformierbare Klemmbacke und mittels eines zur elastischen Spreizung der Klemmbacke dienenden Spannmittels ist das Knochenfixationselement mit dem Querverbinder lösbar verbindbar. Ferner umfasst die erfindungsgemässe Vorrichtung einen auf dem Längsstab koaxial zur Stabachse verschiebbaren, im wesentlichen zylindrischen Längsstabverbinder mit einer zur Stabachse senkrechten Zentralachse, einem oberen, die Zentralachse schneidenden Ende, einem unteren, die Zentralachse schneidenden Ende und einem zur Zentralachse senkrechten, den Längsstabverbinder durchdringenden Kanal zur Aufnahme des Längsstabes und ein Feststellmittel, welches beispielsweise aus einer Verschlusskappe besteht und am oberen Ende koaxial zur Zentralachse mit dem Längsstabverbinder verschraubbar ist, so dass durch den Längsstabverbinder und die Verschlusskappe der Querverbinder und der Längsstab relativ zueinander lösbar fixierbar sind. Der Querverbinder umfasst in einem Abstand vom Hohlraum eine von der Oberseite zur Unterseite durchgehende Bohrung zur um die Zentralachse rotierbaren Aufnahme des Längsstabverbinders, so dass der Querverbinder bei gelöster Verschlusskappe relativ zur Stabachse des Längsstabes schwenkbar ist.

In der bevorzugten Ausführungsform der erfindungsgemässen Vorrichtung ist die Oberseite des Querverbinders abgesetzt ausgebildet, so dass die Klemmbacke mit eingefügtem Knochenfixationselement und Spannmittel gegenüber dem Längsstab so weit vertieft ist, dass der Querverbinder auch bei maximaler Ausschwenkung der zweiten Achse der Klemmbacke relativ zur ersten Achse des Hohlraumes bezüglich der Stabachse des Längsstabes um 360° rotierbar ist.

Vorzugsweise ist der Hohlraum zur ersten Achse axialsymmetrisch und kugelschichtartig ausgebildet, während die Klemmbacke eine zum Hohlraum komplementäre Form mit einer zweiten Achse aufweist. Die Klemmbacke umfasst eine die zweite Achse schneidende, obere Stirnfläche und eine zur zweiten Achse konzentrische, konische Bohrung mit einem konischen Innengewinde, welche von der oberen Stirnfläche sich verjüngend in die Klemmbacke eindringt. Das Spannmittel ist zur konischen Bohrung komplementär kegelstumpfförmig und mit einem konischen Aussengewinde ausgebildet, so dass durch eindrehen des Spannmittels in das konische Innengewinde die Klemmbacke radial gespreizt und gegen die Wandung des Hohlraumes gepresst wird.

In einer Ausführungsform der erfindungsgemässen Vorrichtung bildet die Klemmbacke den Kopfteil und ist mit dem Knochenfixationselement, insbesondere einem Pedikelhaken einstückig, wodurch sich ein einfacher Aufbau der Vorrichtung erreichen lässt.

In einer anderen Ausführungsform der erfindungsgemässen Vorrichtung bildet ein kegelstumpfförmig ausgebildetes und mit einem konischen Aussengewinde versehenes Spannmittel den Kopfteil und ist mit dem Knochenfixationselement, insbesondere einer Pedikelschraube einstückig. Auch durch diese Ausgestaltung lässt sich ein einfacher Aufbau der erfindungsgemässen Vorrichtung erreichen.

In einer weiteren Ausführungsform der erfindungsgemässen Vorrichtung umfasst der Längsstabverbinder an seinem unteren Ende einen konischen Bund, welcher sich gegen das untere Ende erweitert und mit einem komplementär konischen Innenkonus in der Bohrung an der Unterseite des Querverbinders verkeilbar ist. Dadurch ist eine Sicherung gegen Verdrehung des Querverbinders relativ zum Längsstabverbinder erreichbar.

Der konische Bund kann eine Sternverzahnung umfassen, wodurch eine verstärkte Verdrehsicherung zwischen Längsstabverbinder und Querverbinder erreichbar ist.

In wiederum einer weiteren Ausführungsform der erfindungsgemässen Vorrichtung umfasst der Längsstabverbinder einen Absatz, welcher im montierten Zustand auf der Oberseite des Querverbinders aufliegt, wodurch bei der Implantation der Vorrichtung ein Herausfallen des Längsstabverbinders aus der Bohrung im Querverbinder verhindert wird.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Vorrichtung eine Verbindung zwischen der Klemmbacke mit der Pedikelschraube oder dem Pedikelhaken und dem Längsstab hergestellt wird, welche eine Rotation der Klemmbacke relativ zur Stabachse des Längsstabes gestattet. Durch die Ausführung des Querverbinders mit einer gegenüber der Verbindung Längsstab - Querverbinder vertieften Verbindung zwischen Querverbinder und Klemmbacke ist somit eine Rotation der Klemmbacke relativ zur Stabachse des Längsstabes um 360° möglich. Eine Fixierung des Querverbinders relativ zum Längsstab ist in jeder Position möglich. Nach erfolgter Wirbelsäulenkorrektur kann sich das Knochenfixationselement, beispielsweise die Pedikelschraube oder der Pedikelhaken lateral, medial oder unmittelbar unterhalb des Längsstabes befinden. Gegenüber einer unilateral verschiebbaren Klemmbacke verdoppelt sich die mögliche Korrekturdistanz bei gleicher Implantatdimension.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine Explosionsdarstellung der bevorzugten Ausführungsform der erfindungsgemässen Vorrichtung;
Fig. 2 einen Schnitt durch eine Ausführungsform der erfindungsgemässen Vorrichtung;
Fig. 3 einen Schnitt durch eine weitere Ausführungsform der erfindungsgemässen Vorrichtung; und
Fig. 4 einen Schnitt durch eine andere Ausführungsform der erfindungsgemässen Vorrichtung.

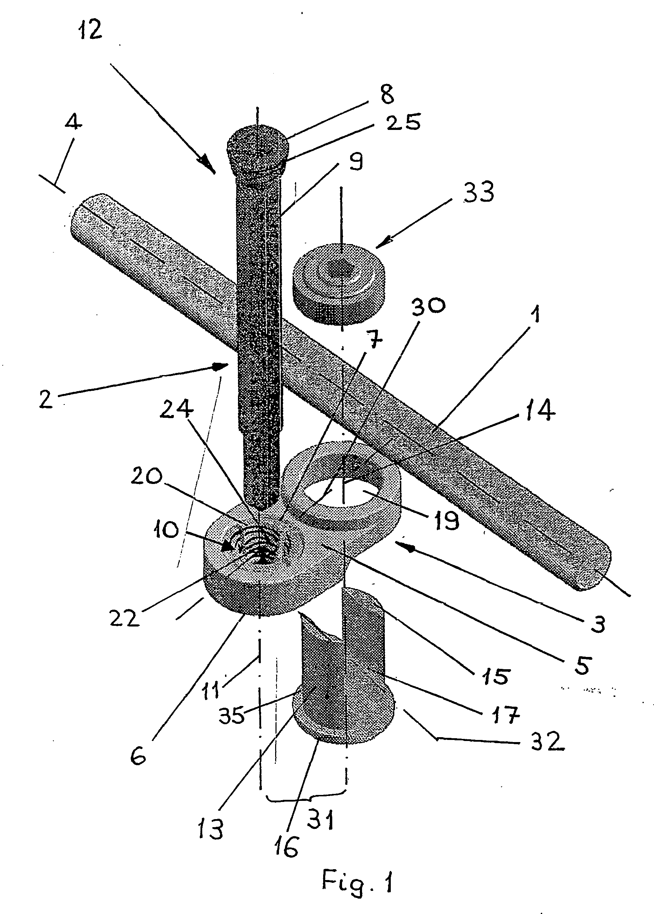

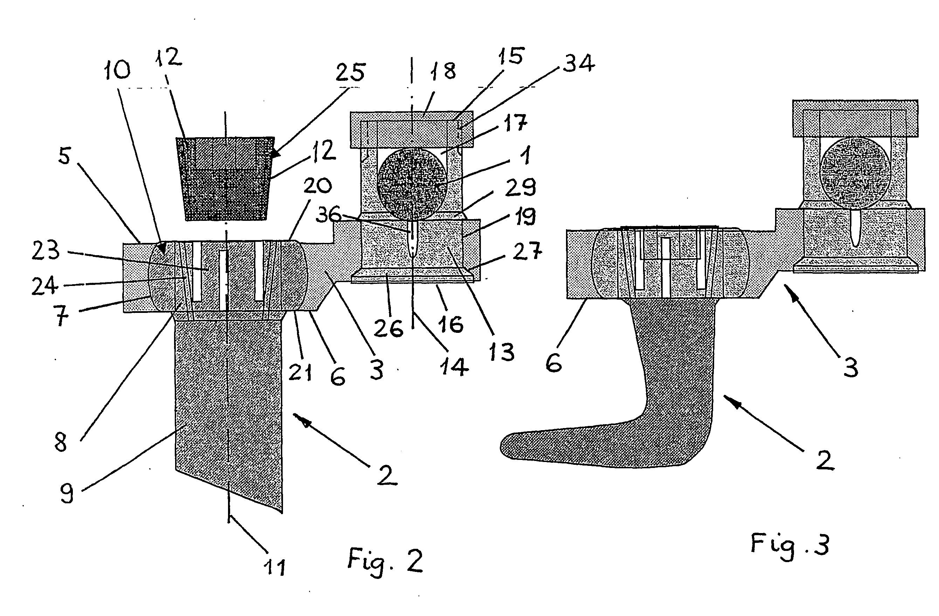

Fig. 1 zeigt die bevorzugte Ausführungsform der erfindungsgemässen Vorrichtung, welche im wesentlichen einen plattenförmigen Querverbinder 3, eine Klemmbacke 10 mit einem Spannmittel 12 zur lösbaren Fixation eines Knochenfixationselementes 2, beispielsweise wie hier gezeigt einer Knochenschraube, und einen Längsstabverbinder 13 mit einem Feststellmittel 33 zur lösbaren Fixation eines Längsstabes 1 am Querverbinder 3. Der Querverbinder 3 ist mit einer Plattenlängsachse 30, einer Plattenoberseite 5 zur Anlage an den Längsstab 1, einer Plattenunterseite 6 und einem den Querverbinder 3 von der Plattenoberseite 5 zur Plattenunterseite 6 koaxial zu einer ersten, im wesentlichen senkrecht auf der Plattenlängsachse 30 stehenden Achse 11 durchdringenden Hohlraum 7 ausgestattet. Ferner umfasst der Querverbinder 3 eine den Querverbinder 3 von der Plattenoberseite 5 zur Plattenunterseite 6 durchdringenden, koaxial zu einer auf der Plattenlängsachse 30 in einem Abstand 31 zur ersten Achse 11 angeordneten und im wesentlichen zur ersten Achse 11 parallelen Zentralachse 14 angeordnete Bohrung 19, welche zur um die Zentralachse 14 rotierbaren Aufnahme des Längsstabverbinders 13 dient. Die Klemmbacke 10 ist zu einer zweiten Achse 22 konzentrisch ausgebildet. Der Hohlraum 7 ist in dieser Ausführungsform zur ersten Achse 11 symmetrisch kugelschichtartig ausgebildet, während die Klemmbacke 10 eine dazu komplementär kugelschichtartige Form aufweist, so dass die Klemmbacke 10 im Hohlraum 7 um die erste Achse 11 sowie um zwei senkrecht dazu stehende Drehachsen schwenkbar aufnehmbar und radial elastisch komprimierbar und dient zur lösbaren Fixation eines Knochenfixationselementes 2 am Querverbinder 3. Das Spannmittel 12, welches zur radialen Spreizung der Klemmbacke 10 und damit zur lösbaren Fixation eines Knochenfixationselementes 2 im Hohlraum 7 dient, bildet den Kopfteil 8 des als Knochenschraube ausgeführten Knochenfixationselementes 2 und ist mit dem Knochenfixationselement 2 einstückig. Die Klemmbacke 10 umfasst eine obere, die zweite Achse 22 schneidende Stirnfläche 20 und eine zur zweiten Achse 22 konzentrische, konische Bohrung 23 mit einem konischen Innengewinde 24, welche von der oberen Stirnfläche 20 in die Klemmbacke 10 eindringt, so dass ein als zur konischen Bohrung 23 komplementär kegelstumpfförmig ausgebildetes und mit einem konischen Aussengewinde 25 versehenes Spannmittel 12 in die konische Bohrung 23 einschraubbar ist, wodurch die Klemmbacke 10 radial gespreizt wird. Bildet beispielsweise der Kopfteil 8 eines als Knochenschraube ausgebildeten Knochenfixationselementes 2 das Spannmittel 12, so wird diese durch Einschrauben des Kopfteiles 8 in die Klemmbacke 10 am Querverbinder 3 lösbar fixiert. Zur Aufnahme des Längsstabes 1 dient ein in der Bohrung 19 koaxial zur Zentralachse 14 aufnehmbarer Längsstabverbinder 13 mit einem, eine dritte, im wesentlichen senkrecht auf der Zentralachse 14 stehende Achse 32 aufweisenden Kanal 17, worin der Längsstab 1 aufnehmbar ist. Der Längsstabverbinder 13 enthält ein oberes, die Zentralachse 14 schneidendes Ende 15 sowie ein unteres, die Zentralachse 14 schneidendes Ende 16 mit einem Bund 35, welcher an der Plattenunterseite 6 zur Anlage bringbar ist. Der Kanal 17 ist gegen das obere Ende 15 des Längsstabverbinders 13 offen. Zur lösbaren Fixation des Längsstabes 1 enthält die erfindungsgemässe Vorrichtung Feststellmittel 33, welche in dieser Ausführungsform als über ein Aussengewinde 34 (Fig. 2) am oberen Ende 15 des Längsstabverbinders 13 schraubbare Verschlusskappe 18 (Fig. 2) ausgebildet sind, wodurch der parallel zur dritten Achse 32 in den Kanal 17 eingelegte Längsstab 1 sowie der Längsstabverbinder 13 relativ zum Querverbinder 3 lösbar fixierbar sind. Bei gelösten Feststellmitteln 33 ist der Querverbinder 3 um die Zentralachse 14 drehbar mit dem Längsstabverbinder 13 angeordnet. Die Oberseite 5 des Querverbinders 3 ist abgesetzt ausgebildet, so dass die Klemmbacke 10 mit eingefügtem Kopfteil 8 eines Knochenfixationselementes 2 und Spannmitteln 12 gegenüber dem Längsstab 1 so weit vertieft ist, dass der Querverbinder 3 bezüglich der Stabachse 4 eines in den Kanal 17 einlegbaren Längsstabes 1 um 360° rotierbar ist.

Die in Fig. 2 dargestellte Ausführungsform der erfindungsgemässen Vorrichtung unterscheidet sich von der in Fig. 1 dargestellten Ausführungsform lediglich darin, dass die Klemmbacke 10 den Kopfteil 8 eines als Knochenschraube ausgebildeten Knochenfixationselementes 2 bildet und mit diesem einstückig ist. Ferner umfasst der Längsstabverbinder 13 an seinem unteren Ende 16 einen konischen Bund 26, welcher sich gegen das untere Ende 16 erweitert und mit einem komplementär konischen Innenkonus 27 in der Bohrung 19 an der Unterseite 6 des Querverbinders 3 verkeilbar ist. Ein Absatz 29 am Längsstabverbinder 13 liegt im montierten Zustand auf der Oberseite 5 des Querverbinders 3 auf. Zur Montage des Längsstabverbinders 13 ist axial ein Schlitz 36 angebracht, wodurch der Absatz 29 so weit radial zusammenpressbar ist, dass der Längsstabverbinder 13 von der Plattenunterseite 6 in die Bohrung 19 einschiebbar ist.

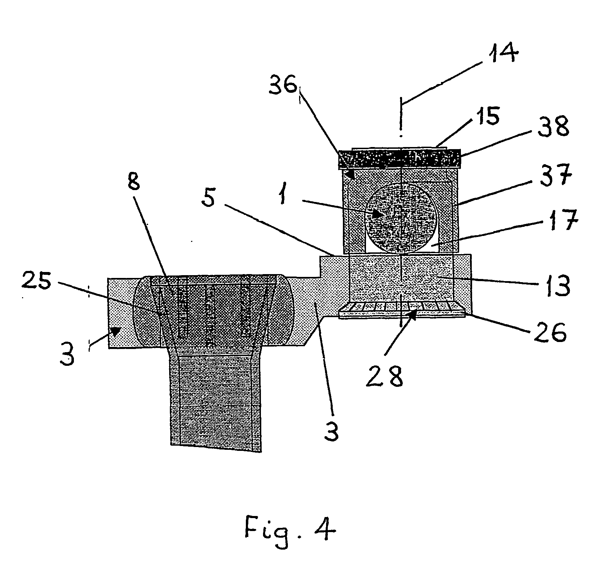

Fig. 3 zeigt eine Ausführungsform der erfindungsgemässen Vorrichtung, die sich von der in Fig. 2 dargestellten Ausführungsform nur darin unterscheidet, dass das Knochenfixationselement 2 anstelle einer Knochenschraube ein Pedikelhaken ist.

In Fig. 4 ist eine Ausführungsform der erfindungsgemässen Vorrichtung gezeigt, welche sich von der unter Fig. 1 beschriebenen Ausführungsform darin unterscheidet, dass der Kanal 17 senkrecht zur Zentralachse 14 an der Mantelfläche des Längsstabverbinders 13 offen ist. Dadurch ist der Längsstab 1 seitlich in den Längsstabverbinder 13 einführbar. Zur seitlichen Fixierung des Längsstabes 1 relativ zum Längsstabverbinder 13 wird eine Hülse 36 mit zwei zur Zentralachse 14 parallelen Seitenwänden 37 vom oberen Ende 15 her über den Längsstabverbinder 13 geschoben und mittels einer über das Aussengewinde 34 schraubbaren Mutter 38 festgezogen. Der Längsstab 1 wird auf diese Weise parallel zur Zentralachse 14 zwischen der Plattenoberseite 5 und der Hülse 36 eingespannt während die Seitenwände 37 eine seitliche Fixation gestatten. Ferner ist der konische Bund 26 mit einer Sternverzahnung 28 versehen, wodurch eine Sicherung gegen Verdrehung um die Zentralachse 14 zwischen Längsstabverbinder 13 und Querverbinder 3 gebildet wird.

## Patentansprüche

1. Vorrichtung zur Verbindung eines Knochenfixationselementes mit einem Längsstab, welche
A) einen plattenförmigen Querverbinder (3) mit einer Plattenlängsachse (30), einer Plattenoberseite (5) zur Anlage an einen Längsstab (1), einer Plattenunterseite (6) und einem den Querverbinder (3) von der Plattenoberseite (5) zur Plattenunterseite (6) koaxial zu einer ersten, im wesentlichen senkrecht auf der Plattenlängsachse (30) stehenden Achse (11) durchdringenden Hohlraum (7);
B) eine zu einer zweiten Achse (22) konzentrische, im Hohlraum (7) aufnehmbare und senkrecht zur zweiten Achse (22) elastisch komprimierbare Klemmbacke (10) zur lösbaren Fixation eines Knochenfixationselementes (2) am Querverbinder (3); und
C) ein Spannmittel (12), welches zur radialen Spreizung der Klemmbacke (10) und damit zur lösbaren Fixation eines Knochenfixationselementes (2) im Hohlraum (7) dient, umfasst,
**dadurch gekennzeichnet, dass**
D)der Querverbinder (3) eine den Querverbinder (3) von der Plattenoberseite (5) zur Plattenunterseite (6) durchdringende, koaxial zu einer die Plattenlängsachse (30) in einem Abstand (31) zur ersten Achse (11) schneidenden und im wesentlichen zur ersten Achse (11) parallelen Zentralachse (14) angeordnete Bohrung (19) umfasst;
E) die Vorrichtung einen in der Bohrung (19) koaxial zur Zentralachse (14) aufnehmbaren Längsstabverbinder (13) mit einem, eine dritte, im wesentlichen senkrecht auf der Zentralachse (14) stehenden Achse (32) aufweisenden Kanal (17) zur Aufnahme eines Längsstabes (1); und
F) Feststellmittel (33) zur lösbaren Fixierung eines koaxial oder parallel zur dritten Achse (32) in den Kanal (17) einlegbaren Längsstabes (1) sowie des Längsstabverbinders (13) relativ zum Querverbinder (3) umfasst, und dass
G) der Querverbinder (3) bei gelösten Feststellmitteln (33) um die Zentralachse (14) drehbar mit dem Längsstabverbinder (13) angeordnet ist.

2. Vorrichtung nach Anspruch 1, **dadurch gekennzeichnet, dass** die Plattenoberseite (5) des Querverbinders (3) abgesetzt ausgebildet ist, so dass die Klemmbacke (10) mit eingefügtem Kopfteil (8) eines Knochenfixationselementes (2) und Spannmitteln (12) gegenüber dem Längsstab (1) so weit vertieft ist, dass der Querverbinder (3) bezüglich der Stabachse (4) eines in den Kanal (17) einlegbaren Längsstabes (1) um 360° rotierbar ist.

3. Vorrichtung nach Anspruch 1 oder 2, **dadurch gekennzeichnet, dass** die Klemmbacke (10) den Kopfteil (8) bildet und mit dem Knochenfixationselement (2) einstückig ist.

4. Vorrichtung nach Anspruch 1 bis 2, **dadurch gekennzeichnet, dass** das Spannmittel (12) den Kopfteil (8) bildet und mit dem Knochenfixationselement (2) einstückig ist.

5. Vorrichtung nach Anspruch 1 bis 4, **dadurch gekennzeichnet, dass** der Hohlraum (7) zur ersten Achse (11) axialsymmetrisch kugelschichtartig ausgebildet ist, die Klemmbacke (10) eine dazu komplementär kugelschichtartige Form aufweist, so dass die Klemmbacke (10) im Hohlraum (7) um zwei senkrecht zur ersten Achse (11) stehende Achsen und um die zweite Achse (22) rotierbar ist.

6. Vorrichtung nach Anspruch 5, **dadurch gekennzeichnet, dass** die Klemmbacke (10) eine obere, die zweite Achse (22) schneidende Stirnfläche (20) und eine zur zweiten Achse (22) konzentrische, konische Bohrung (23) mit einem konischen Innengewinde (24) umfasst, welche von der oberen Stirnfläche (20) in die Klemmbacke (10) eindringt, das Spannmittel (12) zur konischen Bohrung (23) komplementär kegelstumpfförmig mit einem konischen Aussengewinde (25) ausgebildet ist, so dass durch Eindrehen des Spannmittels (12) in die konische Bohrung (23) die Klemmbacke (10) radial spreizbar ist.

7. Vorrichtung nach einem der Ansprüche 1 bis 6, **dadurch gekennzeichnet, dass** der Längsstabverbinder (13) ein oberes, die Zentralachse (14) schneidendes Ende (15) sowie ein unteres, die Zentralachse (14) schneidendes Ende (16) umfasst und der Kanal (17) gegen das obere Ende (15) des Längsstabverbinders (13) offen ist.

8. Vorrichtung nach einem der Ansprüche 1 bis 6, **dadurch gekennzeichnet, dass** der Kanal (17) senkrecht zur Zentralachse (14) offen ist.

9. Vorrichtung nach einem der Ansprüche 1 bis 8, **dadurch gekennzeichnet, dass** der Längsstabverbinder (13) an seinem unteren Ende (16) einen konischen Bund (26) umfasst, welcher sich gegen das untere Ende (16) erweitert und mit einem komplementär konischen Innenkonus (27) in der Bohrung (19) an der Unterseite (6) des Querverbinders (3) verkeilbar ist.

10. Vorrichtung nach Anspruch 9, **dadurch gekennzeichnet, dass** der konische Bund (26) eine Sternverzahnung (28) umfasst, wodurch eine Verdrehsicherung zwischen Längsstabverbinder (13) und Querverbinder (3) gebildet wird.

11. Vorrichtung nach einem der Ansprüche 1 bis 10, **dadurch gekennzeichnet, dass** der Längsstabverbinder (13) einen Absatz (29) umfasst, welcher im montierten Zustand auf der Plattenoberseite (5) des Querverbinders (3) aufliegt.

## Claims

1. An apparatus for connecting a bone fastener to a longitudinal rod, comprising
A) a plate-like transverse coupler (3) with a longitudinal axis (30) a top surface (5) designed to be in contact with a longitudinal rod (1), a bottom surface (6), and a cavity (7) penetrating the transverse coupler (3) from the top surface (5) to the bottom surface (6) and extending coaxially to a first axis (11) arranged essentially vertically to the longitudinal axis (30);
B) a gripping jaw (10) extending concentrically to a second axis (22) which may be received in the cavity (7) and is resiliently compressible vertically to the second axis (22) for releasably fixating a bone fastener (2) on the transverse coupler (3); and
C) a tension means (12) which serves for radially spreading apart the gripping jaw (10) and thus for releasably fixating a bone fastener (2) within the cavity (7),
**characterized in that**
D) the transverse coupler (3) comprises a bore (19) penetrating the transverse coupler (3) from the top surface (5) to the bottom surface (6) and arranged coaxially to a central axis (14) intersecting the longitudinal axis (30) at a distance (31) from the first axis (11) and extending essentially parallel to the first axis (11);
E) the apparatus comprises a longitudinal rod coupler (13) which may be received in the bore (19) in such a way that it extends coaxially to the central axis (14) and which includes a channel (17) with a third axis (32) extending essentially vertically to the central axis (14) wherein a longitudinal rod (1) may be received; and
F) blocking means (33) for releasably fixating, relative to the transverse coupler (3), a longitudinal rod (1) insertable in such a way into the channel (17) that it extends coaxially or parallel to the third axis (32), and the longitudinal rod coupler (13), and that
G) with the blocking means (33) released, the transverse coupler (3) is in rotatable engagement with the longitudinal rod coupler (13).

2. An apparatus as claimed in claim 1, **characterised in that** the top surface (5) of the transverse coupler (3) is offset so that the gripping jaw (10) with the head portion (8) of a bone fastener (2) inserted therein and with tension means (12) is recessed with respect to the longitudinal rod (1) to such an extent that the transverse coupler (3) is rotatable by 360 degrees relative to the rod axis (4) of a longitudinal rod (1) insertable into the channel (17).

3. An apparatus as claimed in claim 1 or 2, **characterised in that** the gripping jaw (10) forms the head portion (8) and that it is realised integral with the bone fastener (2).

4. An apparatus as claimed in claim 1 or 2, **characterised in that** the tension means (12) forms the head portion (8) and that it is realised integral with the bone fastener (2).

5. An apparatus as claimed in any of the claims 1 to 4, **characterised in that** the cavity (7) is shaped in the form of a spherical segment between two parallel circles extending axially symmetrically to the first axis (11), the gripping jaw (10) being shaped in the form of a complementary spherical segment, so that the gripping jaw (10) is rotatable within the cavity (7) about two axes extending vertically to the first axis (11) and about the second axis (22).

6. An apparatus as claimed in claim 5, **characterised in that** the gripping jaw (10) comprises a top end face (20) intersecting the second axis (22) and a conical bore (23) extending concentrically to the second axis (22) and including a conical internal screw thread (24) which penetrates the gripping jaw (10) from the top end face (20), that the tension means (12) is shaped in a frustoconical form complementary to the conical bore (23) and is equipped with a conical external screw thread (25), so that by screwing the tension means (12) into the conical bore (23) the gripping jaw (10) may be radially spread apart.

7. An apparatus as claimed in any of the claims 1 to 5, **characterised in that** the longitudinal rod coupler (13) comprises a top end portion (15) intersecting the central axis (14) and a bottom end portion (16) intersecting the central axis (14), and that the channel (17) is open towards the top end portion (15) of the longitudinal rod coupler (13).

8. An apparatus as claimed in any of the claims 1 to 6, **characterised in that** the channel (17) is open in a direction vertical to the central axis (14).

9. An apparatus as claimed in any of the claims 1 to 8, **characterised in that** the longitudinal rod coupler (13) comprises a conical flange (26) located in its bottom end portion (16) which widens towards said bottom end portion (16) in such a way that it may become wedged in a complementary inner cone (27) formed in the bore (19) on the bottom surface (6) of the transverse coupler (3).

10. An apparatus as claimed in claim 9, **characterised in that** the conical flange (26) comprises a radial toothing (28) whereby the longitudinal rod coupler (13) and the transverse coupler (3) are secured against twisting.

11. An apparatus as claimed in any of the claims 1 to 10, **characterised in that** the longitudinal rod coupler (13) comprises a recess (29) which rests on the top surface (5) of the transverse coupler (3) once the two elements have been put together.

## Revendications

1. Dispositif pour relier un élément de fixation osseuse à une tige longitudinale, comprenant
A) un élément de liaison transversale en forme de plaque (3) ayant un axe longitudinal de plaque (30), une face supérieure de plaque (5) pour l'appui contre une tige longitudinale (1), une face inférieure de plaque (6) et un espace creux (7) traversant l'élément de liaison transversale (3) de la face supérieure de plaque (5) à la face inférieure de plaque (6) coaxialement à un premier axe (11) essentiellement perpendiculaire à l'axe longitudinal de plaque (30) ;
B) une mâchoire de serrage (10), concentrique par rapport à un deuxième axe (22), pouvant être logée dans l'espace creux (7) et pouvant être comprimée de manière élastique perpendiculairement au deuxième axe (22), pour fixer de manière amovible un élément de fixation osseuse (2) sur l'élément de liaison transversale (3) ; et
C) un moyen de contrainte (12) qui sert à écarter radialement la mâchoire de serrage (10) et fixer ainsi de manière amovible un élément de fixation osseuse (2) dans l'espace creux (7),
**caractérisé en ce que**
D) l'élément de liaison transversale (3) comprend un alésage (19) traversant l'élément de liaison transversale (3) de la face supérieure de plaque (5) à la face inférieure de plaque (6), disposé coaxialement à un axe central (14) coupant l'axe longitudinal de plaque (30) à une certaine distance (31) du premier axe (11) et essentiellement parallèle au premier axe (11) ;
E) le dispositif comprend un connecteur de tige longitudinale (13), pouvant être logé dans l'alésage (19) coaxialement à l'axe central (14), qui comprend un canal (17), présentant un troisième axe (32) essentiellement perpendiculaire à l'axe central (14), pour loger une tige longitudinale (1) ; et
F) un moyen de blocage (33) pour fixer de manière amovible une tige longitudinale (1), pouvant être insérée dans le canal (17) coaxialement ou parallèlement au troisième axe (32), ainsi que le connecteur de tige longitudinale (13) par rapport à l'élément de liaison transversale (3),
et **en ce que**
G) l'élément de liaison transversale (3) est disposé de manière à pouvoir tourner autour de l'axe central (14) conjointement avec le connecteur de tige longitudinale (13) lorsque les moyens de blocage (33) sont desserrés.

2. Dispositif selon la revendication 1, **caractérisé en ce que** la face supérieure de plaque (5) de l'élément de liaison transversale (3) est conçue avec un épaulement, de sorte que la mâchoire de serrage (10), avec la partie de tête (8) d'un élément de fixation osseuse (2) insérée et des moyens de contrainte (12), est tellement en retrait par rapport à la tige longitudinale (1) que l'élément de liaison transversale (3) peut tourner à 360° par rapport à l'axe de tige (4) d'une tige longitudinale (1) pouvant être insérée dans le canal (17).

3. Dispositif selon la revendication 1 ou 2, **caractérisé en ce que** la mâchoire de serrage (10) forme la partie de tête (8) et est formée d'une seule pièce avec l'élément de fixation osseuse (2).

4. Dispositif selon la revendication 1 ou 2, **caractérisé en ce que** le moyen de contrainte (12) forme la partie de tête (8) et est formé d'une seule pièce avec l'élément de fixation osseuse (2).

5. Dispositif selon les revendications 1 à 4, **caractérisé en ce que** l'espace creux (7) est réalisé en forme de segment sphérique axisymétrique au premier axe (11), la mâchoire de serrage (10) a une forme de segment sphérique complémentaire de sorte que la mâchoire de serrage (10) peut tourner, dans l'espace creux (7), autour de deux axes perpendiculaires au premier axe (11) et autour du deuxième axe (22).

6. Dispositif selon la revendication 5, **caractérisé en ce que** la mâchoire de serrage (10) comprend une face frontale supérieure (20) coupant le deuxième axe (22) et un alésage conique (23), concentrique par rapport au deuxième axe (22), qui présente un taraudage conique (24), lequel pénètre depuis la face frontale supérieure (20) dans la mâchoire de serrage (10), le moyen de contrainte (12) étant réalisé en forme de cône tronqué avec un filetage conique (25) complémentaire à l'alésage conique (23), de sorte qu'un vissage du moyen de contrainte (12) dans l'alésage conique (23) permet d'écarter radialement la mâchoire de serrage (10).

7. Dispositif selon l'une quelconque des revendications 1 à 6, **caractérisé en ce que** le connecteur de tige longitudinale (13) comprend une extrémité supérieure (15) coupant l'axe central (14) ainsi qu'une extrémité inférieure (16) coupant l'axe central (14), et le canal (17) est ouvert sur l'extrémité supérieure (15) du connecteur de tige longitudinale (13).

8. Dispositif selon l'une quelconque des revendications 1 à 6, **caractérisé en ce que** le canal (17) est ouvert perpendiculairement à l'axe central (14).

9. Dispositif selon l'une quelconque des revendications 1 à 8, **caractérisé en ce que** le connecteur de tige longitudinale (13) comprend, au niveau de son extrémité inférieure (16), un collet conique (26) qui s'élargit en direction de l'extrémité inférieure (16) et qui peut être calé dans l'alésage (19) avec un cône intérieur complémentaire (27) au niveau de la face inférieure (6) de l'élément de liaison transversale (3).

10. Dispositif selon la revendication 9, **caractérisé en ce que** le collet conique (26) comprend une denture en étoile (28) formant une protection anti-torsion entre le connecteur de tige longitudinale (13) et l'élément de liaison transversale (3).

11. Dispositif selon l'une quelconque des revendications 1 à 10, **caractérisé en ce que** le connecteur de tige longitudinale (13) comprend un épaulement (29), lequel connecteur, à l'état monté, s'appuie sur la face supérieure de plaque (5) de l'élément de liaison transversale (3).
